(19) Europäisches Patentamt
European Patent Office
Office européen des brevets

(11) **EP 1 765 097 B1**

(12) **EUROPEAN PATENT SPECIFICATION**

(45) Date of publication and mention
of the grant of the patent:
**04.12.2013 Bulletin 2013/49**

(51) Int Cl.:
*A23L 1/00* (2006.01)   *A23L 1/05* (2006.01)
*A23P 1/00* (2006.01)   *A61B 5/00* (2006.01)

(21) Application number: **05749183.9**

(22) Date of filing: **01.06.2005**

(86) International application number:
**PCT/CA2005/000849**

(87) International publication number:
**WO 2005/117617 (15.12.2005 Gazette 2005/50)**

(54) **INDEX AND METHOD OF USE OF ADAPTED FOOD COMPOSITIONS FOR DYSPHAGIC PERSONS**

INDEX UND VERFAHREN ZUR VERWENDUNG VON ANGEPASSTEN NÄHRMITTELN FÜR SCHLUCKBEHINDERTE

INDICE ET PROCEDE D"UTILISATION DE COMPOSITIONS ALIMENTAIRES ADAPTEES POUR PERSONNES DYSPHAGIQUES

(84) Designated Contracting States:
**AT BE BG CH CY CZ DE DK EE ES FI FR GB GR HU IE IS IT LI LT LU MC NL PL PT RO SE SI SK TR**

(30) Priority: **01.06.2004 US 575373 P**

(43) Date of publication of application:
**28.03.2007 Bulletin 2007/13**

(73) Proprietor: **PROPHAGIA INC.**
**Québec H9X 1Y9 (CA)**

(72) Inventors:
• **DUFRESNE, Thérèse**
**Montréal, Québec H3T 1H8 (CA)**
• **HOUJAIJ, Nada**
**Ville Saint-Laurent, Québec H4L 5B5 (CA)**
• **LACHANCE, Nicole**
**Laval (Québec) H7X 2N5 (CA)**

(74) Representative: **Fiorucci, Hélène et al**
**Cabinet Germain & Maureau**
**12, rue Boileau**
**B.P. 6153**
**69466 Lyon Cedex 06 (FR)**

(56) References cited:
WO-A-01/01789   CA-A1- 2 467 625
US-A- 5 976 084   US-A1- 2004 086 457

• **STAHLMAN LB ET AL: "Perceptual ratings for pureed and molded peaches for individuals with and impaired swallowing" DYSPHAGIA, vol. 16, 2001, pages 254-262, XP002494937**
• **LOBATO-CALLEROS C ET AL: "Use of fat blends in cheese analogs: influence on sensory and instrumental textural characteristics" JOURNAL OF TEXTURE STUDIES, vol. 28, 1997, pages 619-632, XP002494938**
• **CICHERO JAY, MURDOCH BE: "Detection of swallowing sounds: methodology revisited" DYSPHAGIA, vol. 17, 2002, pages 40-49, XP002494939**
• **CICHERO ET AL: 'How Thick is Thick? Multicenter Study of the Rheological and Material Property Characteristics of Mealtime Fluids and Videofluoroscopy Fluids.' DYSPHAGIA. vol. 15, 2000, pages 188 - 200, XP008115278**

**EP 1 765 097 B1**

**Description**

<u>**TECHNICAL FIELD**</u>

The present invention relates to the rheological profile of foods for facilitating the act of swallowing in people suffering of dysphagia. Calculation of texture profiles for solid food substances is determined to overcome the difficulties associated with dysphagia.

<u>**BACKGROUND OF THE INVENTION**</u>

Dysphagia is the inability to swallow or difficulty in swallowing and may be caused by neurological diseases, infections, metabolic diseases or medical interventions. Neurological diseases may be a stroke, Parkinson' disease, amyotrophic lateral sclerosis, brainstem tumors or dementia. Infections may include diphtheria, botulism or syphilis. Metabolic diseases may consist of Cushing's syndrome or thyrotoxicosis. Medical interventions may be side effects of neuroleptic drugs, chemotherapy, surgery or radiation. Swallowing is a complex sequence of actions which is initiated voluntarily and is completed reflexively, whereby food is moved from the mouth through the pharynx and esophagus to the stomach. The act of swallowing occurs in three stages and requires the integrated and coordinated actions of the head and neck structures, involving peripheral sensory input from oropharyngeal afferents and superimposed control from higher nervous systems centers.

In the first phase of normal swallowing, called the oral phase and which is highly voluntary and variable, depending on taste and motivation, food first undergoes preparation. During the oral preparation sequence, food is transformed into a bolus by the action of mastication along with tongue movements, saliva release and mixing. Then, during the transport sequence of the oral phase, the bolus is placed on the surface of the tongue and is subsequently propelled at the back of the mouth into the cavity where both oral and nasal cavities meet, called the pharynx, by the posterior tongue squeezing it against the hard palate with the tongue central groove exhibiting centripetal then centrifugal motion. Close to the time when the bolus reaches the posterior tongue, the second swallowing phase, called the pharyngeal phase, is triggered. During the second phase of normal swallowing, called the pharyngeal phase, a reconfiguration of the pharynx occurs, transforming the oropharynx from a respiratory to a swallowing pathway by opening the inlet to the esophagus and sealing the inlet to the larynx. There is simultaneous apposition of the muscular soft palate to the posterior pharyngeal wall to prevent nasal regurgitation and there is elevation of the larynx, elevation of the hyoid bone and tilting of the arytenoid cartilage to close the airway, thus protecting the lungs against penetration by food material. The elevation of the hyoid bone also pulls open the upper esophageal sphincter. The bolus transport through the pharynx is due to its kinetic energy acquired during the propulsive action of the tongue and by profound shortening of the pharynx, eliminating bolus access to the larynx and propagating pharyngeal contraction. This involves constriction of the walls of the pharynx, backward bending of the epiglottis, and an upward and forward movement of the larynx and trachea. During this phase, respiratory movements are inhibited by reflex. In the third normal swallowing phase, called the esophageal phase, the bolus passes through the opened esophageal sphincter into the proximal esophagus. It then moves down the esophagus into the stomach. This movement is accomplished by momentum from the second phase, peristaltic contractions, and gravity.

Although the main functions of swallowing are the preparation of the bolus and its transfer from the mouth into the stomach, swallowing also serves as a protective reflex for the upper respiratory tract by removing particles trapped in the nasopharynx and oropharynx, returning materials refluxed from the stomach into the pharynx, or removing particles propelled from the upper respiratory tract into the pharynx.

Swallowing dysfunction or dysphagia greatly increases the risk of undernutrition and dehydration, aspiration, choking and therefore is associated with high morbidity, mortality and cost. Estimates of the prevalence of dysphagia in the elderly range from 10% to 22 % and are up to 70 % among residents admitted in long term care institutions.

Actually the clinical management of dysphagia is still an inexact science and is not based on hard evidence supporting the efficacy of any strategy in improving the nutritional status of dysphagic persons. Current best clinical practices to improve most common impaired aspects of swallowing and thus increase oral food and fluid intake involve modification of diet and eating behavior and swallowing therapy techniques.

Application of swallowing therapies other than compensatory postural and dietary therapies, such as supersupraglottic swallow, supraglottic swallow, Mendelsohn maneuver, strengthening exercises and thermal stimulation, require adequate cognitive competency so that the patient can understand and execute directions. This cognitive requirement excludes the majority of persons with neurogenic dysphagia.

In terms of treatment efficacy to counter undernutrition secondary to dysphagia and its high morbidity/mortality levels, the strongest evidence-based recommendation that is made to clinicians involved in the treatment of dysphagia pertains to diet modification.

Texture modification of solids has been suggested to facilitate bolus formation and swallowing. The diet requirements are currently, among others, expressed as soft, minced or pureed foods. The desired texture is usually obtained with a blender or a food processor. The addition of a liquid is frequently required to produce a pureed product that is smooth and without lumps or big particles. However this dilution technique is thought to reduce the nutrient density. Also, the resulting products have been qualified by many as not appealing and bland. Subsequently, there is a decreased food intake and an increased prevalence of undernutrition in the dysphagic population. Special efforts are constantly being made to improve the taste, the appearance and the nutritional value of modified texture foods. Reshaping modified texture foods is a route being explored by few at present. The description of the texture modified diets is usually qualitative.

Dysphagia diets usually take the form of lists of forbidden and allowed foods. They use descriptive terms such as sticky, smooth, soft or homogeneous to characterize these foods. This list of terms creates semantic discrepancies in the clinical management of the diets offered to the dysphagic persons. All dysphagia diets published are mainly based on a descriptive evaluation of the texture of solids and liquids and very little is said about the therapeutic efficacy or quantitative textural characteristics of the foods permitted for the persons. Clinical trials evaluating specifically the efficacy of the various dysphagia diets and quantification of the textural parameters of a nutritious minced or pureed diet are not known.

Many professionals such as doctors, nurses, radiologists, speech-language pathologists, occupational therapists, physiotherapists and dietitians may be required to participate in the clinical evaluation of the dysphagic individual. The multidisciplinary approach required for the treatment of dysphagia necessitates communication and coordination. It is essential to insure that what is clinically observed as a problem during the evaluation of the person is what is conveyed via the dietetic prescription. It is believed that dysphagic individuals able to handle specific test material during clinical evaluations such as bedside examinations and videofluoroscopy should be able to swallow foods of similar texture. Thereafter, a qualitative description of the appropriate foods is given and a subjective evaluation of what the prescribed diet should be is done. A lack of objectivity in the transmission of the clinical information could lead to clinical errors.

Although treatment and diagnosis of dysphagia have been addressed, there is little standardization among health professionals for the nutritional treatment of dysphagia.

CA 2 467 625 A1 is about a method of preparing adapted foods where the compositions allow the facilitation of the acts of eating or drinking for dysphagic patients. The method comprises modulating the parameters of the rheological profile, consisting of firmness, cohesiveness, springiness, gumminess, chewiness and consistency.

WO 01/01789 A1 discloses a gelled nutritional composition which may provide a nutritional supplement, for example for dysphagia people. The composition contains gelled protein: a carbohydrate source, minerals and vitamins. This document deals with a method of providing nutrition to a patient suffering from dysphagia, said method comprising administering the gelled nutritional composition.

The publication Stahlmann LB et al, Dysphagia 2001; 16:254-262 deals with a study whose objective was to determine if there were differences in the ratings of food attributes and ease of chewing and swallowing puree consistencies between participants with normal and impaired swallowing. Food molds have been created to alter the appearance of pureed foods by making the pureed consistencies look more like typically prepared foods.

US 2004/0086457 is about an orally ingestible baked radiopaque product having a dry food texture that can be utilized for testing dysphagia, said product comprising a fat, a sweetener, a moisturizing agent, x-ray contrast agent, flour and optionally flavoring agent.

With respect to foods allowed in dysphagia diets, no single or combination of measurable quantitative parameters has yet been identified to account for clinical efficacy and to exclude the forbidden foods. It would be highly desirable to be provided with an exclusive correlation between certain objective and measurable parameters and their clinical efficiency for dysphagic persons.

## SUMMARY OF THE INVENTION

One object of the present invention, which is defined by the claims, is the ability to identify exclusive texture profile values at which foods are clinically efficient for the treatment of dysphagia, wherein the texture profile or the texture profile at serving temperature is quantified by a Swallowing Texture (ST) index calculated by a mathematical equation consisting of the variables firmness (F), adhesiveness (A), springiness (S) and cohesiveness (C) and expressed as such:

$$\text{ST index} = (F + |A|) \times S \times C$$

Another object of the present invention is the ability to use these exclusive texture profile values for the standardization and control of food formulations necessary in nutritional treatment of dysphagia.

Also related is a diagnostic method using standardized food compositions to evaluate the swallowing capacity of a person. The method is comprised of administering a portion of food composition, having ST index or SSTI index as defined herein, to an individual and measuring the capacity of swallowing, which can be defined as for example, but not limited to, the swallowing time, transit time, or the mastication pattern, average volume per swallow (ml), average time (s) per swallow and swallowing capacity (ml/s), the number of swallows required per bolus, accumulation of food particles in the mouth between deglutitions (mL), fatigue during eating, mastication delay-effort (N), time delay between bites (s), respiratory pattern during swallow, voice pattern and quality after swallowing, clearing of airways, drooling of material outside the mouth (dribble), absence/presence of premature flow in the pharynx, or regurgitation of food through the nasal cavity.

The expression "ST index" as used herein, which is the Swallowing Texture Index, is the result of a mathematical equation and is used herein to quantify texture profiles of food compositions applied specifically to the process of swallowing in humans.

The acronym "TPA" stands for Texture Profile Analysis and is composed of one or more rheological parameters described above.

The acronym "ST" stands for Swallowing Texture.

The acronym "$SSTI_{max}$" stands for Maximum Safe Swallowing Texture Index. It is the maximum value beyond which the texture is no longer considered easy for swallowing for a dysphagic person.

The texture profile at serving temperature of a food composition is adapted for the treatment of dysphagia and prepared with a pureed food substance which may consist of a meat, fish, poultry, vegetable, fruit, baked good, dairy product or a combination of two or more, and is quantified by a ST index which is less than the Maximum Safe ST Index ($SSTI_{max}$), the latter being preferably of 34 at serving temperature.

Exceptions exist for baked goods served cold and having a melting point below 37°C, for which the ST index value may exceed the $SSTI_{max}$ of 34 at serving temperature as long as their ST index at 23°C is below 34.

Also, the texture profile at serving temperature of a food composition adapted for overcoming of dysphagia-related problems and prepared with a minced food substance which may consist of a meat, fish, poultry, vegetable, fruit, baked good, dairy product or a combination of two or more quantified with a ST index less than the $SSTI_{max}$ the latter being preferably of 80 at serving temperature.

In relation to the present invention, there is provided a method to control the transformation of an edible composition used for the oral intake of drugs to correspond to a specific measurable texture profile.

In relation to the present invention, there is provided adapted food compositions, namely pureed and minced foods, which have a demonstrated clinical efficacy, for facilitating the act of swallowing in dysphagic persons.

For the purpose of the present invention the following terms are defined below:

The term "swallowing" as used herein is intended to mean the transit of food substance from lips to stomach including deglutition.

The term "dysphagia" is a swallowing impairment and may occur during the acts of mastication, bolus formation, its deglutition and its transfer, or a combination thereof. "Dysphagia" may be used interchangeably with swallowing disorder or deglutition disorder.

The term "facilitate" and "facilitation", are used herein to mean the compensation for an impaired functioning of the acts of mastication, bolus formation, its deglutition and its transfer or a combination thereof.

The term "firmness" as used herein is intended to mean the force required to obtain a deformation of a body. The firmness measurement unit is expressed here in Newtons. A Newton is a unit of force equal to the force that produces an acceleration of one meter per squared second of a mass of one kilogram. The terms firmness and hardness can be used interchangeably.

The term "cohesiveness" as used herein is intended to mean the strength of the internal bonds making up the body of the food. It can be defined as the molecular force between particles within a body or substance that acts to unite them. Cohesiveness is a ratio of two firmness measurements. Therefore, it has no units.

The term "springiness" as used herein is intended to mean the rate at which deformed foods go back to their original undeformed state after removal of the force. The measurement unit of springiness is expressed here in percentage. The springiness is the property of a substance that enables it to change its length, volume, or shape in direct response to a force affecting such a change and to recover its original form upon the removal of the force. The terms springiness and elasticity can be used interchangeably.

The term "adhesiveness" as used herein is intended to mean the force necessary to overcome the attractive forces between the surface of a matter and the surface of another material with which it is in contact. The adhesiveness is the attractive molecular force that tends to hold together unlike bodies when they are in contact. The measurement unit of adhesiveness is expressed here in Newtons.

The term " gumminess" is defined as the energy required to disintegrate a food product. It is related to the primary parameters of firmness and cohesiveness (respectively F and C). It is expressed in Newtons.

The term " chewiness" is defined as the energy required to masticate a food product It is related to the primary parameters of firmness, cohesiveness and springiness (F.C.S). It is expressed in Newtons.

The expression "melting point" is the temperature at which a solid becomes a liquid at normal atmospheric pressure.

The expression "therapeutic pureed food" as used herein is intended to mean an adapted food composition with the following characteristics:

A food substance selected from the group consisting of a pureed meat, fish, poultry, vegetable, fruit, baked good, pastry, egg, dairy product or a combination of two or more ;

has a particle size of generally less than about 0.5mm spheric ;

homogeneous and generally absent of syneresis in the plate and in the mouth under pressure of the utensil or tongue ;

may be shaped in order to be appetizing, often in a shape comparable to the not transformed counterpart ;

served hot or cold, generally at the same temperature as the not transformed counterpart ;

facilitates swallowing, especially in persons with a severely impaired oral phase and/or pharyngeal phase.

The expression "therapeutic minced food" as used herein is intended to mean an adapted food composition with the following characteristics:

a food substance selected from the group consisting of a minced meat, fish, poultry, vegetable, fruit, baked good, pastry, egg, dairy product or a combination of two or more ;

has a particle size of generally between about 2mm to 5mm spheric ;

homogeneous and generally absent of syneresis in the plate and in the mouth under pressure of the utensil or tongue ;

may be shaped in order to be appetizing, often in a shape comparable to the not transformed counterpart ;

served hot or cold, generally at the same temperature as the not transformed counterpart ;

facilitates swallowing, especially in persons with a mildly impaired preparation sequence of the oral phase.

The acronym "SAH" stands for Ste. Anne's Hospital

## BRIEF DESCRIPTION OF THE DRAWINGS

Figs. 1(a) and 1(b) illustrates the normal swallow and dysphagia in the elderly respectively;

Fig. 2 illustrates a flowchart representing work progression of the experimental process;

Fig. 3 shows a Texture Profile Analysis: Force deformation curve schematic of a food sample showing a first and second compression;

Figs. 4a to 4f illustrate bar graphs of six different textural parameters for each different food sub-group; and

Fig. 5 shows a graphical representation of $SSTI_{max}$ for therapeutic pureed and therapeutic minced food products.

## DETAILED DESCRIPTION OF THE PREFERRED EMBODIMENT

The present invention is defined by the claims and will be described more fully hereinafter with reference to the accompanying drawings, in which preferred embodiments of the invention are shown.

One embodiment of the present invention is to provide a method in which a quantitative and descriptive approach is used to adapt the food texture in the clinical management of dysphagia. A description of textural characteristics of foods is provided and is prone to be an integral part of the clinical management of dysphagia. No known prior art has reported quantified solid food texture in relation to its definition in the health care of dysphagic individuals. Rheology is the study of the deformation and flow of matter. It offers vocabulary and specific terminology to discuss foods and their textural characteristics. Rheology needs utilization of several instruments such as viscometers, consistometers and texturometers which permit quantification of these textural characteristics.

In accordance with the present invention, there is provided a new index and method of use thereof for determining and modulating the physical characteristics of solid foods. For two principal classes of therapeutic foods, for example, but without limiting it to, therapeutic minced and therapeutic pureed, physical states are provided. The method of the invention allows the application of an integrated combination of all the simple texture parameters quantified by the ST index. The Swallowing Texture (ST) index is calculated by a mathematical equation consisting of the variables firmness (F), adhesiveness (A), springiness (S) and cohesiveness (C) and expressed as such:

$$ST\ index = (\ F + |\ A\ |\ )\ x\ S\ x\ C$$

In accordance with the present invention, there are provided two new Maximum Safe Swallowing Texture Indices ($SSTI_{max}$) calculated with the new ST index formula which represent the thresholds of clinical efficiency for the nutritional treatment of dysphagia. These two new indices apply to two principal groups of therapeutic foods: purees and minced products.

One particular utility of the method of the present invention is the possibility to prepare standardized batches of food adapted for persons having different outstanding traits of swallowing dysfunctions.

The method of the present invention is based on the determination of a new index, the ST index, which integrates and comprises the modulation of at least one parameter of a food's texture profile in a manner to allow the food composition at serving temperature to have a desired combination of firmness, adhesiveness, springiness and cohesiveness.

Another aspect of the method of the present invention is to serve as an index for food description and categorization having useful applications in various health sectors such as nutrition, geriatrics, dentistry and pediatrics.

The method comprised also provides a standardized combination of therapeutic foods as described herein. A person's swallowing ability can be first evaluated for indications of dysphagia when the subject swallows the composition having a first ST index. The person's swallowing ability can then be evaluated further for indications of dysphagia when the subject swallows the compositions having other ST indices. Because the compositions are of known and standardized ST indices, far more useful information is generated allowing generally accurate diagnoses, generally efficient treatments and generally uniformized understandings of diet prescriptions.

The utilities of the standardized compositions and methods are several fold. A primary utility is optimizing the textural profiles of those standardized compositions in order to facilitate deglutition according to various levels of swallowing disorders and consequently to counter undernutrition subsequent to dysphagia. Another utility is that by using standardized compositions, consistency in treating dysphagia is promoted. Rather than supplying dysphagic persons an arbitrarily modified texture food, the subject is supplied a composition of known and standardized physical characteristics.

The present invention provides a method allowing the transformation of food substances into a food composition characterized by a certain ST index to facilitate the act of swallowing for dysphagic persons. [*] [*] The present invention provides a method for the preparation of a food composition for improving swallowing in a dysphagic person according to anyone of claims 1 to 7.

In relation to the present invention, there is provided a method allowing the diagnosis of the presence and degree of dysphagia in a patient. Food compositions having different ST and SSTI indexes are prepared and administered to tested patients. Different parameters are then measured to assess the dysphagia severity or presence of the residual swallowing capacity. For example, but not limited to, the swallowing time, transit time, or the mastication pattern, average volume per swallow (ml), average time (s) per swallow and swallowing capacity (ml/s), the number of swallows required per bolus, accumulation of food particles in the mouth between deglutitions (mL), fatigue during eating, mastication delay-effort (N), time delay between bites (s), respiratory pattern during swallow, voice pattern and quality after swallowing, clearing of airways, drooling of material outside the mouth (dribble), absence/presence of premature flow in the pharynx, regurgitation of food through the nasal cavity can be measured for this aspect.

Also important is whether the problem is difficulty swallowing or pain on swallowing (odynophagia). Odynophagia may suggest inflammatory or malignant neoplastic processes. The level of sensation of the difficulty in swallowing ("the catch") should be sought. Suprasternal pain suggests a hypopharyngeal location of disease. A substernal or subxyphoid location of symptoms suggests an esophageal source. These locators can be misleading, though, as distal esophageal problems can occasionally present with suprasternal discomfort.

The administering of the food composition according to the present invention could allow to assess the severity of dysphagia and could allow to discriminate between different types and reasons of dysphagia, and offer different alternatives for overcoming transient or permanent swallowing difficulties. For example, dysphagia to solids may suggest esophageal or other structural obstruction. Dysphagia to liquids may suggest pharyngeal disorders, including neuromuscular disease. These data can be combined with the observation of weight loss in a patient with dysphagia, which is an indicator of the significance and duration of the disease. Also combined to dietary changes in response to the dysphagia, this will give insight to the person skilled in the art into the nature and severity of disease.

Using this test, swallowing function can be qualified and quantified on a ratio scale and expressed, for example but not limited to, as percent of residual swallowing capacity,; such information may improve the predictive value of clinical assessment and provides a practical way of monitoring change in patients with dysphagia.

The present invention will be more readily understood by referring to the following example which is given to illustrate the invention rather than to limit its scope.

## EXAMPLE 1

**Texture and Sensorial Evaluation of Foods Specialized for the Treatment of Dysphagia**

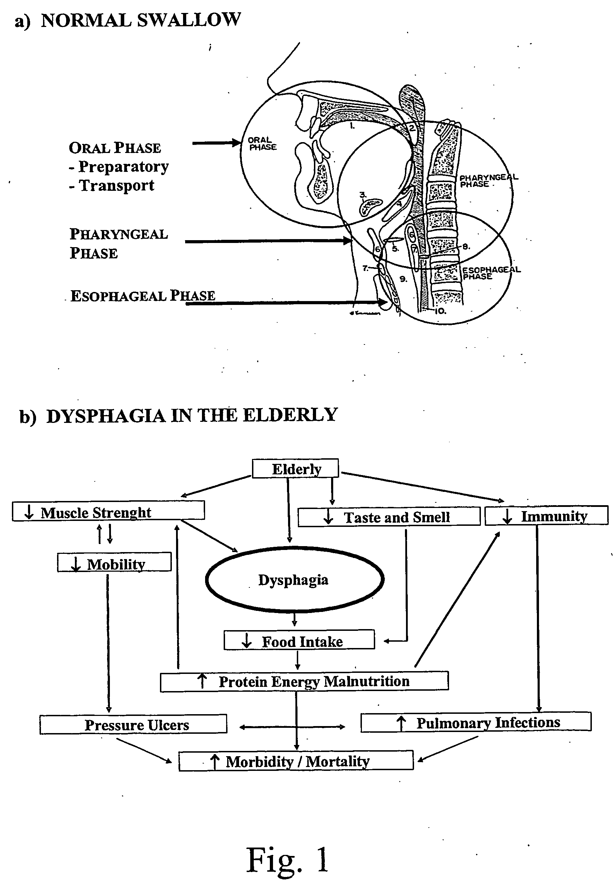

A database was established consisting of 67 pureed and 30 minced foods for which both evaluations for clinical efficiency and rheological analyses were done (Table 1). Quantitatively speaking, a texture profile analysis, also known as TPA, was obtained for each food sample. These TPAs provide valuable information about the texture of a food product and give us some insight as to how a food product reacts to the pressure or force applied by jaw, tongue and cheeks during eating. The equipment used to measure TPA attempts to simulate the preparation of the bolus of phase 1 described by Groher, M.E. (Dysphagia: Diagnosis and Management. 3rd ed. Boston: Butterworth-Heinemann, 1997. 338 p) (Figs. 1a & 1b). This simulation compresses the food sample and measures the textural parameters which are of concern to us. Foods were grouped according to their composition - pureed or minced - and their respective textural parameters were separated and statistically analyzed.

## Table 1

### Database of pureed and minced therapeutic food products used for sensorial textural analyses

| | | Type of food | No. of Samples |
|---|---|---|---|
| Purees | | Meats, poultry, fish | |
| | | Meats, meat dishes | 27 |
| | | Poultry | 5 |
| | | Fish | 4 |
| | | Sub-total | 36 |
| | | Vegetables and fruits | |
| | | Vegetables | 9 |
| | | Fruit | 3 |
| | | Sub-total | 12 |
| | | Baked goods | |
| | | Cakes @ 12°C | 6 |
| | | Cakes @ 23°C | 13 |
| | | Sub-total | 19 |
| | | Total Purees | 67 |
| Minced | | Meat, poultry, fish | |
| | | Meat, meat dishes | 27 |
| | | Poultry | 3 |
| | | Total Minced | 30 |
| | | Total Database | 97 |

One objective achieved through this experiment was the development of a new method and formula to quantify TPA in order to identify Safe ST index zones for therapeutic pureed and minced food product formulations.

From a qualitative point of view, food samples were also sensorially evaluated in this experiment by a clinical expert in the treatment of dysphagia who provided essential descriptions of food texture. These analyses were made to link quantitative values to qualitative descriptions and consequently associations were deduced between the condition of a food product in the mouth and scientifically validated quantitative values measured by the texture machine.

### Methodology

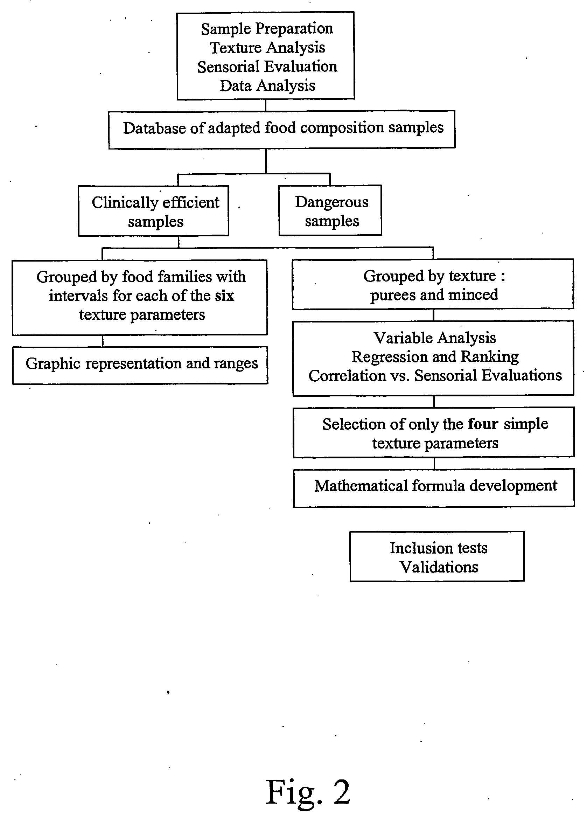

The work progression of the experimental process is illustrated in the flow chart of Fig. 2.

Using rheological instrumental methods, quantitative evaluations of therapeutic minced and pureed foods were performed to provide a better understanding of their textural characteristics. Universal Testing Machine or a texture meter (Lloyd Model LRX, Fareham, Hans U.K.) was used to measure the textural attributes such as firmness, cohesiveness, springiness, adhesiveness, chewiness and gumminess. It was fitted with a 50N load cell and a 50mm diameter disk-shaped probe at a speed of 25mm/min to carry out a two-bite compression test on each food sample. Samples were 3cm x 3cm x 2cm and were individually heated and tested at normal serving temperatures for example, 65°C for meats and vegetables and 23°C or 12°C for cakes and fruits. Rcontrol Data Analysis Software (version 3.2, 1995) gathered the desired textural data using a personalized program. It is pertinent to note that the testing environment of this texture meter is at room temperature by opposition to qualitative evaluations done at a body temperature of 37°C.

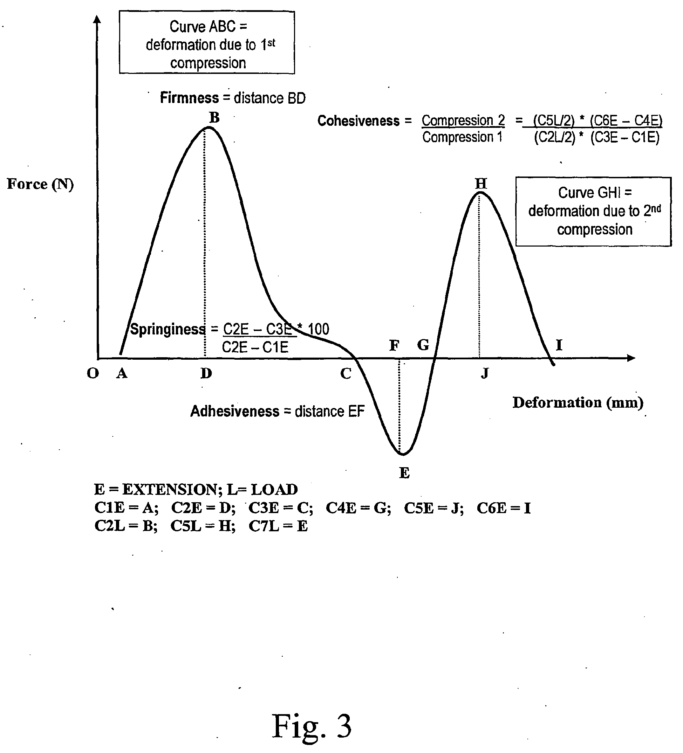

A schematic of the force deformation curve is shown in Fig. 3 from which the textural parameters are derived as indicators of the textural properties which can be divided into two main categories: primary mechanical characterisitics and secondary mechanical characteristics as defined previously.

Evaluation sheets were handed out to our clinical expert taster and were designed with a specific purpose of having a complete and comprehensive description for each food item as pertaining to its textural feel in the mouth, clinical efficacy, diet application and other organoleptic factors. Simultaneously, TPAs were performed on each of these food items in order to correlate between the quantitative values of the textural parameters (TPA described earlier) and their qualitative descriptions according to the clinical expert. Subsequently, ST index values were recorded for each. Food descriptors retained included: compaction or lack of homogeneity or heterogeneity of particle size, cohesiveness, syneresis and adhesiveness. Furthermore, samples were classified as being clinically excellent, acceptable, mediocre or dangerous. The excellent and acceptable samples were retained in the database as being clinically efficient while the dangerous were retained in the database as being clinically not efficient. From hereon, the final database was established.

## Results

Firmness, adhesiveness, springiness and cohesiveness serve as basic essential quantifiers of texture directly related to the swallowing process whereas gumminess and chewiness are derived from the latter. It was observed that a directly proportional relationship existed between the parameters of firmness alone and firmness and springiness versus gumminess and chewiness respectively. For every increase in the firmness of a therapeutic food product, a corresponding increase was evident in terms of gumminess. The same trend appeared for decreased firmness in certain therapeutic foods. Similarly, the increased or decreased effect of one or both of the firmness and springiness parameters produced the exact same result for chewiness.

Hence, gumminess and chewiness being complex textural products calculated from the simple textural parameters, their effects were considered not pertinent and our efforts were concentrated on the changes taking place within firmness, adhesiveness, springiness and cohesiveness.

The four main parameters of concern were firmness, adhesiveness, springiness and cohesiveness.

Firmness was a parameter of concern because it affects the force required to bite, to masticate, to compress the bolus with the tongue and push it back through the back of the mouth into the pharynx (Fig. 3). It is the slope calculated when x = 0 of the ascending curve drawn by the texture meter (Fig. 3).

$$F = BD = C2L \hspace{3cm} \text{Equation (1)}$$

where F = firmness

Adhesiveness was a parameter of concern because it affects the energy which is required to overcome the attractive forces between the food composition and the structures of the oral cavity, such as the tongue and hard and soft palate (Fig. 3). It is the negative of the slope after the first compression and before the second compression calculated when x = 0 of the ascending curve drawn by the texture meter (Fig. 3).

$$A = EF = C7L \hspace{3cm} \text{Equation (2)}$$

where A = adhesiveness

Springiness was a parameter of concern because it affects the ability of a food composition to return to its original shape after being compressed by the actions of mastication and compression of the tongue on the hard palate (Fig. 3).

$$S = \frac{C2E - C3E}{C2E - C1E} * 100 \hspace{3cm} \text{Equation (3)}$$

where S = springiness

Cohesiveness was a parameter of concern because it affects the attractive force required to hold together the molecules of a food composition. A certain cohesion range was identified facilitating bolus transportation, bolus deglutition and allowing shaping of pureed and minced foods. Cohesiveness is an important parameter for the esthetic appearance of the food through its shaping capabilities of holding the food matter together. From a psychological point of view, this enhanced attractive value of food presentation is highly desirable especially among elderly dysphagic persons on a rigorous puree diet (Fig.3).

$$C = \text{Compression 2 / Compression 1} \hspace{3cm} \text{Equation (4)}$$

where C = cohesion, Compression 1 = (C2L/2) x (C3E - C1E), and Compression 2 = (C5L/2) x (C6E - C4E).

Pureed food samples evaluated as "clinically excellent" and as "clinically acceptable" by the clinical expert were considered "clinically efficient" for patients with a severely impaired oral and/or pharyngeal phase of swallowing.

Minced food samples evaluated as "clinically excellent" and as "clinically acceptable" by the clinical expert were considered "clinically efficient" for patients with a mildly impaired preparation sequence of the oral phase of swallowing.

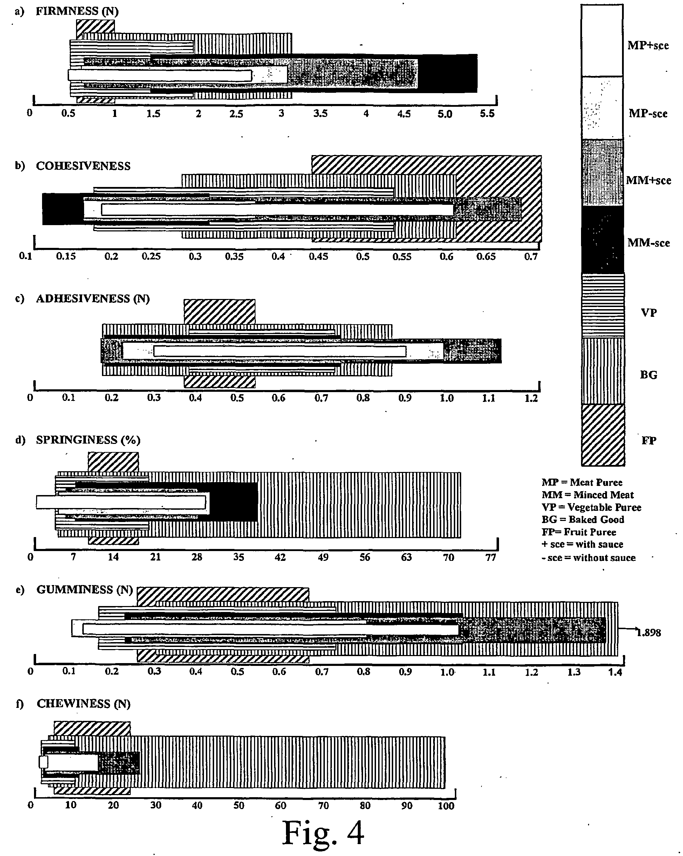

Minimal and maximal values were calculated for each textural parameter for each food sample in the database. For clinically efficient samples, their ranges for firmness, adhesiveness, springiness and cohesiveness were recorded and were reported as follows (Table 2). When grouped by therapeutic food families, it was observed that firmness, adhesiveness, springiness and cohesiveness values varied considerably (Figs. 4a to 4f). Over the course of time, many venues were followed to attempt to clearly explain the observed trends in shifting values of all the textural parameters for each therapeutic food sample related directly to each of their sensorial evaluations compiled throughout our experiment with our clinical expert taster. The objective was to bring together the relative effect of each parameter into a ST index demonstrating the combined overall effect of all four parameters of firmness, adhesiveness, springiness and cohesiveness into one mathematical equation with specifically assigned numeric values.

To determine a Maximum Safe ST Index ($SSTI_{max}$), the rheological values of the clinically non-efficient samples were used. The $SSTI_{max}$ represented the upper limits, respectively for both pureed and minced foods, below which, all clinically efficient samples should fit. Several formulas were analyzed through various mathematical manipulations for which the clinical efficacy was reflected by the result of the formula. Inclusion tests were performed for each formula in order to show the number of samples which do not fit below its $SSTI_{max}$ (Table 3).

**Table 2**

| Ranges in firmness, adhesiveness, springiness and cohesiveness for clinically efficient pureed and minced foods | | | | |
|---|---|---|---|---|
| **Textural Parameter** | **Purees N=62** | | **Minced N=29** | |
| | **Min.** | **Max.** | **Min.** | **Max.** |
| **Firmness** | 0,452 | 7,566 | 0,679 | 5,330 |
| **Adhesiveness** | -0,164 | -0,978 | - 0,163 | - 1,102 |
| **Springiness** | 3,054 | 71,661 | 0,117 | 0,679 |
| **Cohesiveness** | 0,159 | 0,718 | 5,148 | 47,446 |

**Table 3**

| Non-exhaustive list of inclusion tests for fitting a correlation between clinical efficacy and numerical results | | | |
|---|---|---|---|
| **Test #** | **Equation** | **Results of inclusion test** | |
| | | **Purees N=62 # of unfit** | **Minced N=29 # of unfit** |
| **1** | F*A*S*C | 5 | 0 |
| **2** | (F-|A|)*C/S | 13 | 11 |
| **3** | (F-|A|)/(S*C) | 18 | 21 |
| **4** | (F-|A|)+(C/S) | 0 | 2 |
| **5** | (F-|A|)*(S/100)*C | 5 | 0 |
| **6** | (F-|A|)+C+(S/100) | 1 | 1 |
| **7** | ((F-|A|)+(S/100))/C | 1 | 7 |
| **8** | ((F-|A|)+((S/100)*C) | 0 | 2 |
| **9** | ((F-|A|)+(S/100))*C | 5 | 0 |
| **10** | ((F+|A|)+(S/100))*C | 3 | 1 |

(continued)

| Non-exhaustive list of inclusion tests for fitting a correlation between clinical efficacy and numerical results | | | |
|---|---|---|---|
| Test # | Equation | Results of inclusion test | |
| | | Purees N=62 # of unfit | Minced N=29 # of unfit |
| 11 | (F+|A|)*S*C | 6 | 0 |
| 12 | (F-|A|)+C+S | 43 | 0 |
| 13 | ((F-|A|)+S)/C | 8 | 0 |
| 14 | ((F-|A|)+(S*C) | 14 | 1 |
| 15 | ((F-|A|)+S)*C | 17 | 1 |
| 16 | ((F+|A|)+S)*C | 0 | 1 |
| 17 | (F+|A|)+((S/100)/C) | 0 | 2 |

The preferable fit formula according to an inclusion test was at zero exclusion for both purees and minced samples. One equation, test # 11, presented this result of zero exclusion with the exception of all six pureed cake samples at 12°C (Tables 3 & 4). These exceptions will be discussed later.

### Table 4

| Inclusion test for fitting a correlation between clinical efficacy numerical results when number of unfit for minced is 0. | | | | |
|---|---|---|---|---|
| Test # | Equation | Results of inclusion test | | |
| | | Purees N=62 # of unfit | Minced N=29 # of unfit | Outlying cakes 12°C |
| 5 | (F-|A|)*(S/100)*C | 5 | 0 | 5 |
| 9 | ((F-|A|)+(S/100))*C | 5 | 0 | 4 |
| 11 | (F+|A|)*S*C | 6 | 0 | 6 |
| 12 | (F-|A|)+C+S | 43 | 0 | 6 |
| 13 | ((F-|A|)+S)/C | 8 | 0 | 4 |

The formula used for Test # 11 takes into account the combined effect of all forces being applied to the food sample when placed in the mouth and undergoing two consecutive compressions. The formula used for Test # 11 is (Firmness + |Adhesiveness|) x Springiness x Cohesiveness:

$$F + |A|) \text{ x S x C} \qquad\qquad\qquad\qquad \text{Equation (5)}$$

In the first part of the Equation (5), exists a cumulative effect consisting of all vertical forces represented in Fig. 3. These include the firmness which is a positive displacement force and the adhesiveness which is a negative displacement force. The absolute value of the latter is taken into account for calculating the additive impact of these forces playing a role in breaking apart both the internal bonds holding the particles of the food sample together and the bonds responsible for allowing the food material to adhere to the interior of the mouth including the teeth, tongue and palate. Both firmness and adhesiveness are forces expressed in Newtons.

The second part of the equation accounts for both the springiness and cohesiveness effects which ultimately influence the transformation of the food sample into a bolus and its subsequent transportation from the mouth back into the pharynx. Springiness represents the ability of food to resist the forces being applied to it and to return it to its original form once the deforming forces have been removed. Cohesiveness, on the other hand, maintains the integrity of the food structure from within and keeps it from degrading too quickly under the effect of mastication, tongue compression and/or salivation. Once breakdown of internal food bonds is initiated, cohesiveness decreases sharply. Hence, the combined effect of the resistance of a food to breakdown, expressed by the addition of its firmness and adhesiveness and multiplied by its springiness and cohesiveness defines the ease of a food sample to form into a bolus and to be moved backwards into the pharynx.

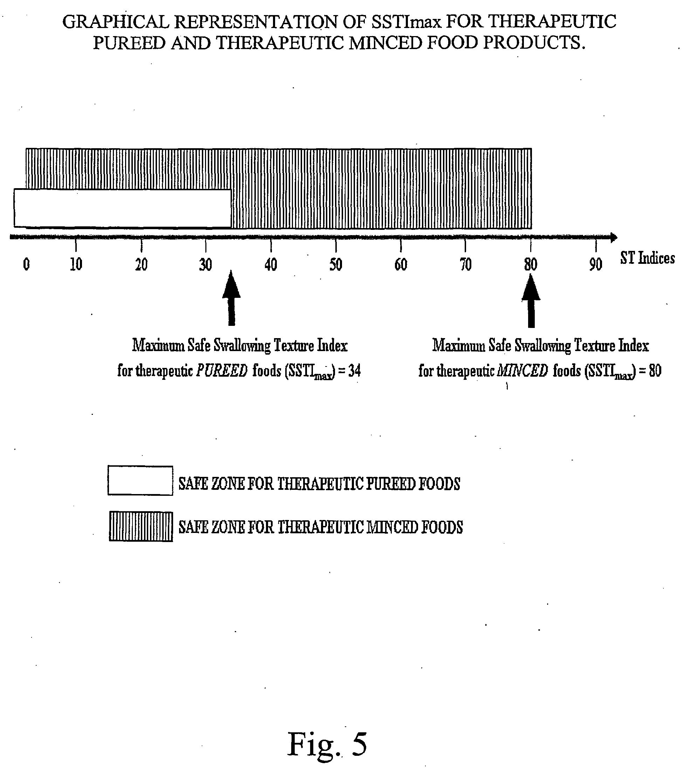

The $SSTI_{max}$ calculated by Equation (5), showed a value of 34 below which pureed foods are clinically efficient (Fig. 5).

The $SSTI_{max}$ calculated by Equation (5), showed a value of 80 below which minced foods are clinically efficient (Fig. 5).

However, a certain exception exists for the pureed baked goods group whereby the $SSTI_{max}$ established for purees does not necessarily apply. All therapeutic pureed cake samples served cold, show a discrepancy between their positive clinical efficiency and their negative result on the inclusion test with a $SSTI_{max}$ established by Equation (5). An explanation for this discrepancy is the fact that all cake samples were formulated by using a regular domestic type gelatin which is heat sensitive and has a melting point of 32°C.

Once these pureed cakes come in contact with a warm environment, represented in this case by the temperature of the mouth and tongue at 37°C, the gelatin will significantly rise in temperature and almost immediately may change from one phase into another rendering it softer and subsequently more easily broken down in the mouth. Indeed, all of these pureed cake samples, when measured at 23°C, had a ST index below 34.

**Claims**

1. A method for the preparation of a food composition for improving swallowing in a dysphagic person, said method comprising the steps of :

   a) processing a solid food composition to obtain a minced solid food composition,
   b) quantifying at serving temperature the Swallowing Texture Index (ST index) of the solid food composition, wherein said ST index is calculated according to the formula:

   $$\text{ST index} = (F + \left| A \right|) \times S \times C$$

   wherein the parameters are the following :

   F is firmness and is expressed in Newtons, I A | is absolute value of adhesiveness and is expressed in Newtons, S is springiness and is expressed in percentage and C is cohesiveness,
   wherein the ST Index quantified in step b) is less than or equal to 80.

2. The method of claim 1, wherein said solid food composition is selected from the group consisting of meat, fish, poultry, vegetable, fruit, baked good, dairy product and a combination thereof.

3. The method of claim 1 or claim 2, wherein said processing further comprises at least one of the following actions: adding a food texture modifier, crunching, grinding, chopping, mixing, blending, stirring, incorporating a gas, warming, heating, cooking, cooling, refrigerating, freezing, retherming, diluting, applying pressure, modifying the particle size and creating a new macro-structure within of said solid food composition.

4. The method according to claim 3, wherein said food texture modifier is selected from the group consisting of a binding, a gelling and a thickening compound.

5. The method according to anyone of claims 3 or 4, wherein said food texture modifier is selected from the group consisting of a protein, a carrageenan, a starch, a fiber, an alginate, a pectin, a gum, a gelatin, and a combination thereof.

6. A method for the preparation of a food composition for improving swallowing in a dysphagic person, said method comprising the steps of:

   a) processing a solid food composition to obtain a pureed solid food composition,
   b) quantifying at serving temperature the Swallowing Texture Index (ST index) of the solid food composition, wherein said ST index is calculated according to the formula:

$$ST\ index = (F + \left|A\right|)\ x\ S\ x\ C$$

wherein the parameters are the following :

F is firmness and is expressed in Newtons, |A| is absolute value of adhesiveness and is expressed in Newtons, S is springiness and is expressed in percentage and C is cohesiveness, wherein the ST Index quantified in step b) is less than or equal to 34.

7. The method of claim 6, wherein said solid food composition is selected from the group consisting of meat, fish, poultry, vegetable, fruit, baked good, dairy product and a combination thereof.

**Patentansprüche**

1. Verfahren zur Herstellung einer Lebensmittelzusammensetzung zur Verbesserung der Schluckfähigkeit bei einer Person, die an Dysphagie leidet, wobei das Verfahren die folgenden Schritte umfasst:

a) Verarbeiten einer festen Lebensmittelzusammensetzung, um eine zerhackte feste Lebensmittelzusammensetzung zu erhalten,
b) Quantifizieren bei einer Vorlegetemperatur des Schluck-Struktur-Indexes (ST-Index) der festen Lebensmittelzusammensetzung, wobei der genannte ST-Index gemäß der folgenden Formel errechnet wird:

$$ST\ index = (F + \left|A\right|)\ x\ S\ x\ C$$

wobei die Parameter wie folgt sind:

F ist die Stabilität und ist in Newton ausgedrückt, |A| ist der absolute Wert der Klebfestigkeit und ist in Newton ausgedrückt, S ist die Elastizität und ist in einem Prozentsatz ausgedrückt und C ist die Kohäsion, wobei der ST-Index, der in Schritt b) quantifiziert ist, weniger als oder gleichwertig 80 beträgt.

2. Verfahren nach Anspruch 1, wobei die genannte feste Lebensmittelzusammensetzung ausgewählt ist aus der Gruppe bestehend aus Fleisch, Fisch, Geflügel, Gemüse, Obst, Backwaren, Milchprodukten sowie einer Kombination davon.

3. Verfahren nach Anspruch 1 oder Anspruch 2, wobei die genannte Verarbeitung ferner wenigstens eine der folgenden Aktionen umfasst: das Hinzufügen eines Lebensmittelstruktur-Modifizierers, Zermahlen, Pulverisieren, Hacken, Mischen, Vermischen, Rühren, Einarbeiten eines Gases, Erwärmen, Erhitzen, Kochen, Abkühlen, Kühlstellen, Gefrieren, Aufwärmen, Verdünnen, Beaufschlagen von Druck, Modifizieren der Partikelgröße und das Erzeugen einer neuen Makro-Struktur innerhalb der genannten festen Lebensmittelzusammensetzung.

4. Verfahren nach Anspruch 3, wobei der genannte Lebensmittelstruktur-Modifizierer ausgewählt ist aus der Gruppe bestehend aus einer Binde-, einer Gelier- und einer Verdickungsverbindung.

5. Verfahren nach einem der Ansprüche 3 oder 4, wobei der genannte Lebensmittelstruktur-Modifizierer ausgewählt ist aus der Gruppe bestehend aus einem Protein, einem Carrageenan, einer Stärke, einer Faser, einem Alginat, einem Pektin, einem Gummi, einer Gelatine sowie einer Kombination davon.

6. Verfahren zur Herstellung einer Lebensmittelzusammensetzung zur Verbesserung der Schluckfähigkeit bei einer Person, die an Dysphagie leidet, wobei das Verfahren die folgenden Schritte umfasst:

a) Verarbeiten einer festen Lebensmittelzusammensetzung, um eine pürierte feste Lebensmittelzusammensetzung zu erhalten,
b) Quantifizieren bei einer Vorlegetemperatur des Schluck-Struktur-Indexes (ST-Index) der festen Lebensmittelzusammensetzung, wobei der genannte ST-Index gemäß der folgenden Formel errechnet wird:

$$\text{ST index} = (\text{F} + |\text{A}|) \times \text{S} \times \text{C}$$

wobei die Parameter wie folgt sind:

F ist die Stabilität und ist in Newton ausgedrückt, |A| ist der absolute Wert der Klebfestigkeit und ist in Newton ausgedrückt, S ist die Elastizität und ist in einem Prozentsatz ausgedrückt und C ist die Kohäsion, wobei der ST-Index, der in Schritt b) quantifiziert ist, weniger als oder gleichwertig 34 beträgt.

7. Verfahren nach Anspruch 6, wobei die genannte feste Lebensmittelzusammensetzung ausgewählt ist aus der Gruppe bestehend aus Fleisch, Fisch, Geflügel, Gemüse, Obst, Backwaren, Milchprodukten sowie einer Kombination davon.

**Revendications**

1. Procédé pour la préparation d'une composition alimentaire pour améliorer la déglutition chez une personne dysphagique, ledit procédé comprenant les étapes consistant à :

a) traiter une composition alimentaire solide pour obtenir une composition alimentaire solide émincée,
b) quantifier, à la température de consommation, l'indice de texture de déglutition (indice ST) de la composition alimentaire solide, ledit indice ST étant calculé conformément à la formule :

$$\text{indice ST} = (\text{F} + |\text{A}|) \times \text{S} \times \text{C}$$

dans laquelle les paramètres sont les suivants :

F est la fermeté et est exprimée en Newtons, |A| est la valeur absolue de l'adhésivité et est exprimée en Newtons, S est la souplesse et est exprimée en pourcentage, et C est la cohésion, dans lequel l'indice ST quantifié dans l'étape b) est inférieur ou égal à 80.

2. Procédé selon la revendication 1, dans lequel ladite composition alimentaire solide est choisie dans le groupe constitué par la viande, le poisson, la volaille, les légumes, les fruits, les produits de boulangerie, les produits laitiers, et leurs combinaisons.

3. Procédé selon la revendication 1 ou la revendication 2, dans lequel ledit traitement comprend en outre au moins l'une des actions suivantes : addition d'un modificateur de texture alimentaire, fragmentation, broyage, hachage, mélange, combinaison, agitation, incorporation d'un gaz, réchauffage, chauffage, cuisson, refroidissement, réfrigération, congélation, remise à température, dilution, application de pression, modification de la granulométrie, et création d'une nouvelle macrostructure à l'intérieur de ladite composition alimentaire solide.

4. Procédé selon la revendication 3, dans lequel ledit modificateur de texture alimentaire est choisi dans le groupe constitué par un liant, un gélifiant, et un épaississant.

5. Procédé selon l'une quelconque des revendications 3 et 4, dans lequel ledit modificateur de texture alimentaire est choisi dans le groupe constitué par une protéine, une carraghénane, un amidon, une fibre, un alginate, une pectine, une gomme, une gélatine, et une de leurs combinaisons.

6. Procédé pour la préparation d'une composition alimentaire pour améliorer la déglutition chez une personne dysphagique, ledit procédé comprenant les étapes consistant à :

a) traiter une composition alimentaire solide pour obtenir une composition alimentaire solide en purée,
b) quantifier, à la température de consommation, l'indice de texture de déglutition (indice ST) de la composition alimentaire solide, ledit indice ST étant calculé conformément à la formule :

$$\text{indice ST} = (F + |A|) \times S \times C$$

dans laquelle les paramètres sont les suivants :

F est la fermeté et est exprimée en Newtons, |A| est la valeur absolue de l'adhésivité et est exprimée en Newtons, S est la souplesse et est exprimée en pourcentage, et C est la cohésion,
dans lequel l'indice ST quantifié dans l'étape b) est inférieur ou égal à 34.

7. Procédé selon la revendication 6, dans lequel ladite composition alimentaire solide est choisie dans le groupe constitué par la viande, le poisson, la volaille, les légumes, les fruits, les produits de boulangerie, les produits laitiers, et leurs combinaisons.

a) **NORMAL SWALLOW**

ORAL PHASE
- Preparatory
- Transport

PHARYNGEAL
PHASE

ESOPHAGEAL PHASE

b) **DYSPHAGIA IN THE ELDERLY**

## Fig. 1

Sample Preparation
Texture Analysis
Sensorial Evaluation
Data Analysis

Database of adapted food composition samples

Clinically efficient
samples

Dangerous
samples

Grouped by food families with
intervals for each of the six
texture parameters

Grouped by texture :
purees and minced

Graphic representation and ranges

Variable Analysis
Regression and Ranking
Correlation vs. Sensorial Evaluations

Selection of only the **four** simple
texture parameters

Mathematical formula development

Inclusion tests
Validations

# Fig. 2

Curve ABC =
deformation due to 1st
compression

Firmness = distance BD

B

Cohesiveness = $\dfrac{\text{Compression 2}}{\text{Compression 1}}$ = $\dfrac{(C5L/2) * (C6E - C4E)}{(C2L/2) * (C3E - C1E)}$

Force (N)

H

Curve GHI =
deformation due to 2nd
compression

Springiness = $\dfrac{C2E - C3E}{C2E - C1E}$ * 100

F   G                    I

O   A         D              C              J

Deformation (mm)

Adhesiveness = distance EF

E

E = EXTENSION; L= LOAD
C1E = A;   C2E = D;   C3E = C;   C4E = G;   C5E = J;   C6E = I
C2L = B;   C5L = H;   C7L = E

Fig. 3

a) FIRMNESS (N)

b) COHESIVENESS

c) ADHESIVENESS (N)

d) SPRINGINESS (%)

e) GUMMINESS (N)

1.898

f) CHEWINESS (N)

MP+sce
MP-sce
MM+sce
MM-sce
VP
BG
FP

MP = Meat Puree
MM = Minced Meat
VP = Vegetable Puree
BG = Baked Good
FP= Fruit Puree
+ sce = with sauce
- sce = without sauce

## Fig. 4

GRAPHICAL REPRESENTATION OF SSTImax FOR THERAPEUTIC
PUREED AND THERAPEUTIC MINCED FOOD PRODUCTS.

Maximum Safe Swallowing Texture Index
for therapeutic *PUREED* foods (SSTI$_{max}$) = 34

Maximum Safe Swallowing Texture Index
for therapeutic *MINCED* foods (SSTI$_{max}$) = 80

☐ SAFE ZONE FOR THERAPEUTIC PUREED FOODS

▦ SAFE ZONE FOR THERAPEUTIC MINCED FOODS

Fig. 5

**EP 1 765 097 B1**

**Patent documents cited in the description**

- CA 2467625 A1
- WO 0101789 A1
- US 20040086457 A

**Non-patent literature cited in the description**

- **STAHLMANN LB et al.** *Dysphagia,* 2001, vol. 16, 254-262
- **GROHER, M.E.** Dysphagia: Diagnosis and Management. 1997, 338